# EUROPEAN PATENT APPLICATION

(11) **EP 4 755 614 A1**
(43) Date of publication of application: **10.06.2026**
(21) Application number: 25210298.3
(22) Date of filing: 22.10.2025
(51) Int. Cl.: B29C 70/38, G01N 21/84, G01N 33/00

(54) **METHODS FOR AUTOMATED PLY BOUNDARY MARKING**

(30) Priority: 09.12.2024 US 202418974558
(71) Applicant: The Boeing Company, Arlington, VA 22202 (US)
(72) Inventor: HART, Marcus C., Arlington, VA, 22202 (US); FORSTON, Gabriel Z., Arlington, VA, 22202 (US)
(74) Representative: Hoffmann Eitle

(57) **Abstract**

A plurality of fiber tows (214) are laid down (102) by an automated lamination machine (200) (e.g., an automated fiber placement (AFP) machine or a contoured tape laying machine (CTLM)), each fiber tow having a cut end (218). At least one marking (222) is automatically applied within at least one marked region (224) of each respective fiber tow (214). Each marking (222) is configured to provide location information about the respective fiber tow on which the respective marking is placed. The markings are applied using a contrasting color on each respective fiber tow, with the contrasting color being chosen to contrast with a color of the fiber tows such that the markings are configured to enable visual inspection of the plurality of fiber tows. The markings may be designed for human visual inspection, or automated inspection by a machine, and individual fiber tows or plies of fiber tows may be inspected as the tows are being laid down.

## Description

### FIELD

The present disclosure relates generally to composite lamination, and more particularly to methods for automated ply boundary marking.

### BACKGROUND

Automated lamination machines, such as automated fiber placement (AFP) machines and contoured tape laying machines (CTLMs) are often used in the manufacturing of composite parts for automated placement of fiber tows pre-impregnated with resin onto tooling or molds. These automated machines typically place a plurality of separate narrow strips, or tows, next to each other and are meant to increase deposition rates and precision in the production of composite parts. Some automated lamination machines are configured to place courses that are each made up of a plurality of tows of the composite material. For example, some automated lamination machines are configured to place courses each including between 8 and 32 tows, with each tow being between 3 millimeters (mm) and 12 mm wide, though automated lamination machines may be configured to place courses having more or fewer tows in each course, and/or wider or narrower tows. A compaction roller or head of the automated lamination machine can be moved relative to the mold or tooling to steer the tows as they are laid down to provide a desired pattern of the tows and/or courses.

The lamination or stacking of such composite materials by AFP machines and CTLMs requires inspection of ply boundaries to validate that the laid down material is actually located where it is defined in the engineering definition for the part. This typically is accomplished through manual (i.e., human) visual inspection of the material. The human aspect of this inspection operation requires stopping the lamination equipment for a technician to enter the automated equipment cell to perform the inspection. This process is interruptive and time consuming. Prior art attempts to inspect ply boundaries via image analysis have failed to successfully replace manual inspection because the material being inspected tends to be same composition and color as the material below it, and it is challenging to visually inspect a black material on a black substrate or black underlying layer. Laser profilometers have been used to scan fiber tows to determine tow-to-tow gaps, but the resolution of such techniques is limited and current gap sizes are reaching the limits of its resolution.

### SUMMARY

Disclosed methods may enable identifying, with automated or manual inspection, the cut end and fiber direction during material laydown, such as during the laydown of fiber tows by an automated lamination machine such as an automated fiber placement (AFP) machine or a contoured tape laying machine (CTLM). An end effector of the AFP machine or CTLM may be provided with the ability to apply marks (e.g., to spray color or color striping) directly onto the fiber tows during laydown. A control program controlling the AFP machine or CTLM identifies where the material will be cut and then may automatically spray on or otherwise apply a marking in a desired shape (e.g., circular, stripes, etc.) on each fiber tow as it is laid down, thereby creating automated ply boundary markings on the fiber tows during laydown. The marking may be applied in the direction of the longitudinal axis of the fiber and/or may extend up to and past where the material will be cut. Once the material is laid down, or as it is laid down, the ends of each fiber tow and the fiber direction can be inspected using the markings applied to the fiber tows. Such markings may enable determination of tow-to-tow distance and/or course-to-course gaps of the material as it is laid down without requiring the use of a laser profilometer or similar device. In some examples, automated image analysis may be used to complete the inspection of the ply boundary during lamination.

An exemplary method according to the present disclosure may include laying down a plurality of fiber tows and applying at least one marking within at least one marked region of each respective fiber tow of the plurality of fiber tows. The laying down of the plurality of fiber tows may be performed via an automated lamination machine such as an AFP machine or a CTLM, and each respective fiber tow of the plurality of fiber tows may include a respective cut end. Each respective marking may be configured to provide location information about the respective fiber tow on which the respective marking is placed. Applying the at least one marking may include applying a contrasting color onto each respective fiber tow of the plurality of fiber tows, with the contrasting color being configured to contrast with the color of the fiber tows such that the markings are configured to enable visual inspection of the plurality of fiber tows by a human and/or machine.

### BRIEF DESCRIPTION OF THE DRAWINGS

Fig. 1 is a schematic flowchart diagram of methods according to the present disclosure.
Fig. 2 is a schematic black box representation of non-exclusive examples of systems for implementing methods according to the present disclosure.
Fig. 3 is a schematic representation of one ply of fiber tows with markings being positioned to overlay another ply of fiber tows with markings according to the present disclosure.
Fig. 4 is a schematic representation of fiber tows laid down by an automated lamination machine, showing course gap markings and tow gap markings on the fiber tows.
Fig. 5 is a schematic representation of material on a creel that is subsequently cut to form a plurality of marked fiber tows in accordance with the present disclosure.
Fig. 6 is a schematic representation of a plurality of fiber tows, each having a marking bisected by the cut location when the fiber tow is cut, according to the present disclosure.
Fig. 7 is a perspective view of a plurality of courses of material placed on a tooling by an automated lamination machine.
Fig. 8 is a schematic representation of an apparatus that may include one or more composite parts made with markings according to methods of the present disclosure.

### DESCRIPTION

The Figures generally provide illustrative, non-exclusive examples according to the present disclosure. Elements that serve a similar, or at least substantially similar, purpose are labeled with like numbers in each of the Figures, and these elements may not be discussed in detail herein with reference to each Figure. Similarly, all elements may not be labeled in each Figure, but reference numerals associated therewith may be utilized herein for consistency. Elements, components, and/or features that are discussed herein with reference to one or more Figures may be included in and/or utilized with any other of the Figures without departing from the scope of the present disclosure. In general, elements that are likely to be included in a given (i.e., a particular) example are illustrated in solid lines, while elements that are optional to a given example are illustrated in dashed lines. However, elements that are shown in solid lines are not essential to all examples, and an element shown in solid lines may be omitted from a particular example without departing from the scope of the present disclosure.

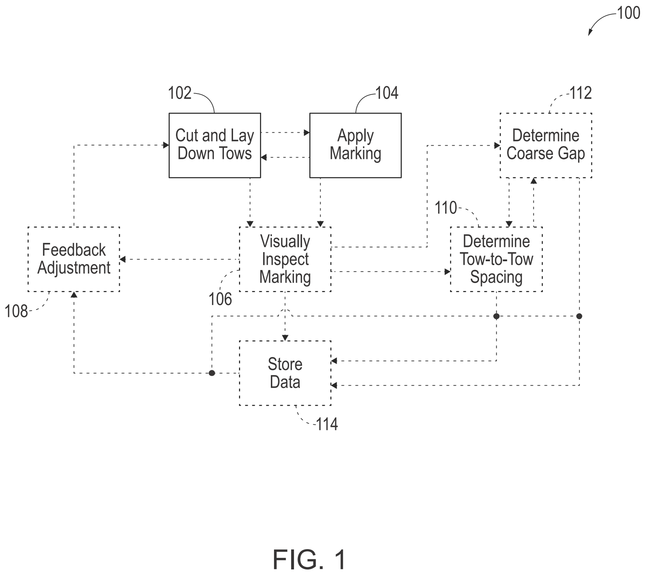

Fig. 1 schematically provides a flowchart that represents illustrative, non-exclusive examples of methods 100 according to the present disclosure. In Fig. 1, some steps are illustrated in dashed boxes indicating that such steps may be optional or may correspond to an optional version of a method according to the present disclosure. That said, not all methods according to the present disclosure are required to include the steps illustrated in solid boxes. The methods 100 and steps illustrated in Fig. 1 are not limiting and other methods and steps are within the scope of the present disclosure, including methods having greater than or fewer than the number of steps illustrated, as understood from the discussions herein.

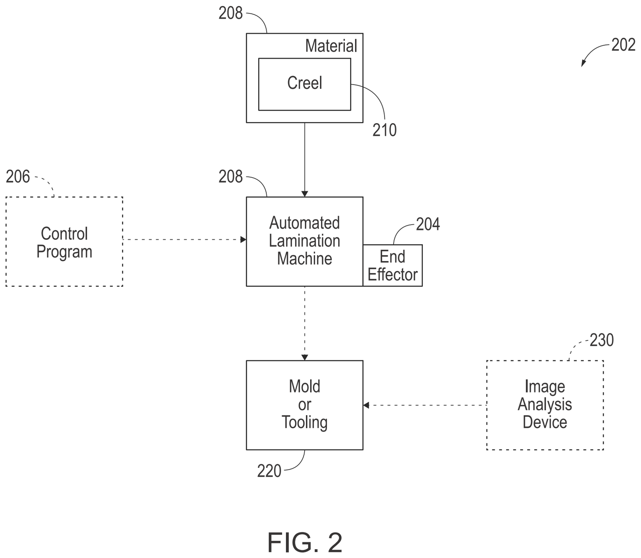

Generally, disclosed methods 100 may enable identifying with automated or manual inspection the cut end and/or fiber direction of fiber tows during material laydown, such as during the laydown of fiber tows by an automated lamination machine 200 (represented schematically in the black box diagram of Fig. 2, which shows systems 202 for implementing disclosed methods 100). As used herein, automated lamination machines may include automated fiber placement (AFP) machines, contoured tape laying machines (CTLMs), flat tape laying machines (FTLMs), and/or other machines configured for automated tape laying or automated fiber placement. An end effector 204 of automated lamination machine 200 may be provided with the ability to apply marks (e.g., to spray color, or apply color striping) directly onto the fiber tows before and/or during laydown. A control program 206 configured to control automated lamination machine 200 identifies where material 208 will be cut (e.g., from a creel 210) and then may automatically cause the automated lamination machine to spray on or otherwise apply a marking in a desired shape (e.g., circular, stripes, etc.) on each fiber tow as it is laid down, thereby creating automated ply boundary markings on the fiber tows during laydown.

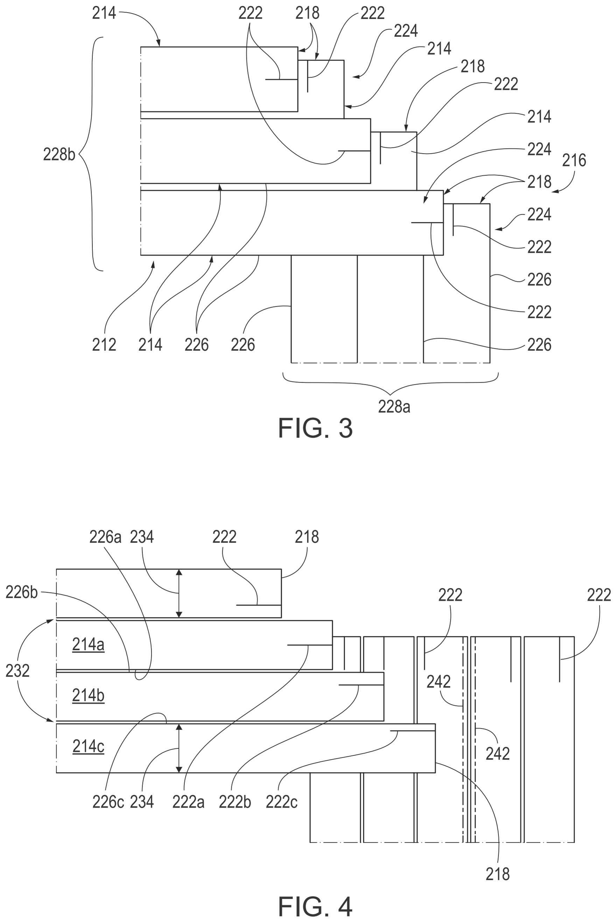

Thus, disclosed methods 100 include laying down a plurality of fiber tows at 102 and applying at least one marking to each respective fiber tow of the plurality of fiber tows at 104. Laying down the plurality of fiber tows at 102 generally is performed by automated lamination machine 200, though the desired markings discussed herein also may be applied during manual layups as well. Fig. 3 schematically represents a second layer, or ply, 212 of fiber tows 214 having been laid on a first ply 216 of fiber tows 214. Each fiber tow 214 has a cut end 218, which is typically cut by automated lamination machine 200 as each fiber tow 214 is laid down (e.g., on a mold, tooling, mandrel, etc. 220). Fig. 3 also shows an example of a marking 222 that may be applied to each fiber tow 214, at 104.

With reference to Figs. 1-3, markings 222 may be applied at 104 within a marked region 224 of each respective fiber tow 214. Each marking 222 is configured to provide location information about the respective fiber tow 214 on which the respective marking 222 is placed. Markings 222 also are configured to enable visual inspection of the plurality of fiber tows 214. In some examples, marked region 224 of some or all of fiber tows 214 is positioned at or adjacent to the respective cut end 218 of the respective fiber tow 214, as shown in Fig. 3 (e.g., cut end 218 may be within marked region 224). The markings applied at 104 may be applied anywhere along the length of each fiber tow (e.g., marked region 224 may be located in other locations of fiber tow 214 in various examples within the scope of the present disclosure). Additionally or alternatively, at least one marked region 224 may be positioned at or adjacent to a respective side edge 226 of a respective fiber tow 214, wherein the respective side edge 226 is substantially perpendicular to the respective cut end 218 of the respective fiber tow 214 in the case of straight fiber tow placement. In the case of steered fiber tow placement, side edges may not be perpendicular to the cut ends, though such steered tows (like straight tows) may include marked regions along one or both side edges, and/or along one or both cut ends of the tow. In this manner, marked region 224 may include ply boundaries and/or cut locations of fiber tows and/or courses laid down by an automated lamination machine.

As noted, laying down the fiber tows at 102 may include laying down the plurality of fiber tows such that the plurality of fiber tows at least partially overlaps an underlying layer. Some automated lamination machines 200 are configured to lay down a plurality of fiber tows at once (e.g., a plurality of fiber tows are laid down in a single pass of the head or end effector 204. In these examples, the plurality of fiber tows 214 laid down together may collectively be referred to as a course 228. For example, Fig. 3 shows a first course 228a and a second course 228b at least partially overlapping first course 228a. First ply 216 may include a plurality of first courses 228a, and/or second ply 212 may include a plurality of second courses 228b. Laying down the fiber tows at 102 may include laying down a plurality of courses (e.g., courses 228) adjacent one another, laying down a plurality of courses on top of one another to create stacked plies formed of individual fiber tows, and/or laying down individual fiber tows next to and/or on top of one another. In some examples, laying down the fiber tows at 102 includes cutting each fiber tow from a spool or creel of material (e.g., creel 210 of material 208), thereby forming each respective cut end of each respective fiber tow.

In some methods 100, applying the markings at 104 is automated. For example, applying the markings at 104 may be performed by automated lamination machine 200. In some examples, applying the at least one marking at 104 is performed automatically by automated lamination machine 200 during the laying down the plurality of fiber tows 214. Laying down the fiber tows at 102 generally is performed according to an engineering definition for a composite part to be formed by the plurality of fiber tows 214.

The markings applied at 104 (e.g., markings 222) may be configured to characterize the fiber tows, course, and/or plies laid down by an automated lamination machine, such as to highlight and differentiate each respective fiber tow from an underlying layer (e.g., to highlight the cut location of the top ply from the underlying layer of previously laminated material), to identify a respective fiber tow from amongst the other fiber tows laid down, to identify the lane of the automated lamination machine that laid down the respective fiber tow, to visualize the cut edge of the fiber tow, to record and/or determine or capture spatial data such as the location of a fiber tow and/or its cut end, and/or to measure or determine course gaps between adjacent courses and/or tow gaps (or overlap) between adjacent fiber tows. For example, markings 222 shown on fiber tows 214 of second ply 212 may be configured to highlight and differentiate each fiber tow of second ply 212 from the respective fiber tows 214 of first ply 216 underlying second ply 212. This generally may be accomplished by using a color for markings 222 that sufficiently contrasts with the color of the material of underlying layer(s) such that the desired fiber tows can be identified via markings 222. Additionally or alternatively, markings 222 may be configured to highlight and differentiate each respective fiber tow 214 from one or more adjacent fiber tows 214 within a given ply 212, 216 or a given course 228. In some examples, applying the at least one marking at 104 includes applying a different respective marking to each respective fiber tow.

In some examples, applying the markings at 104 includes applying at least one marking to each respective fiber tow (e.g., each fiber tow 214), such as by spraying, inking, rolling on, and/or otherwise applying a visual indicator of a contrasting color onto each respective fiber tow. The contrasting color is configured to contrast with the fiber tow on which it is applied and/or with an underlying layer of material and is a color that is preferably easily distinguishable by human vision and/or by image analysis. For example, in the case of tows and plies formed of carbon fiber reinforced polymer composite materials, which typically are black in color, the contrasting color chosen for applying the markings at 104 may be a light and/or bright color, such as shades of white, yellow, orange, or red, though other colors also may provide a sufficient color contrast, such as neon colors, and/or light or bright shades of blue, green, and purple. These colors are examples of colors that may provide a contrast for markings 222, though markings 222 are not limited to these colors. In the case of other materials involving other types of fibers, such as fiberglass or aramid fibers, which typically are lighter in color, the markings applied at 104 may be darker in order to contrast with the lighter color of the fibers, and darker shades of red, blue, green, purple, brown, black, grey, etc., may all be suitable.

In some examples, the fiber tows are pre-impregnated with an un-cured resin that reacts with some materials. Thus, additionally or alternatively, applying the markings at 104 may include applying contrasting materials to the fiber tows that are able to come in contact with any un-cured resin of the fiber tows without chemically reacting with the un-cured resin. For example, non-exclusive examples of suitable materials may include contact approved ink, resin, or another constituent material.

Applying the markings at 104 may be performed by the automated lamination machine (e.g., automated lamination machine 200) in an automated fashion. For example, applying at least one marking at 104 may be automatically performed as each respective fiber tow is laid down by the automated lamination machine. In some examples, applying at least one marking at 104 is performed before material used to form the plurality of fiber tows is placed onto a spool or creel (e.g., before material 208 is loaded or placed onto creel 210) configured for use with the automated lamination machine, and/or before the material on the spool or creel is cut to create each fiber tow. Applying the markings at 104 includes color striping the fiber tows and/or applying a QR code to each fiber tow, in some examples. QR codes applied to the fiber tows at 104 may be configured to directly connect placement of each respective fiber tow to one or more inspection reports and/or other documentation and/or tie the inspection into the digital thread of a production system. In some examples, applying the at least one marking at 104 includes applying a different respective QR code to each respective fiber tow and/or to each respective ply of fiber tows laid down. In some examples, such QR codes may be used in quality control processes for the finished part.

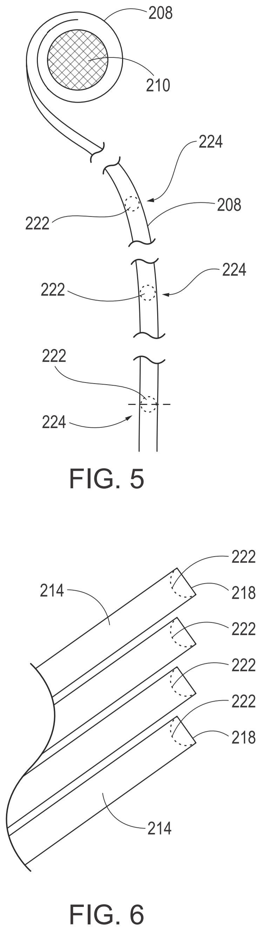

The markings may be applied in the direction of the longitudinal axis of the fiber tow, and may extend up to and past where the material will be cut. Thus, in some methods, applying the markings at 104 includes identifying a respective location of each respective cut end of the material and applying the markings to the material such that each respective marking intersects a respective cut end when the material is cut. For example, Fig. 5 schematically represents material 208 on creel 210, with multiple marked regions 224 on material 208 each having a marking 222 formed in a color that contrasts with the color of material 208. Markings 222 may be placed at any desired location. In some examples, markings 222 are placed in each desired (i.e., predetermined) cut location such that material 208 is cut at the cut locations to form fiber tows 214 (Fig. 6). The desired length of each fiber tow generally is known in advance, such that the spacings between cut ends and/or markings may be known based on the desired length of fiber tows to be laid down. Fig. 6 schematically represents fiber tows 214 that were cut from material 208 of Fig. 5, with each marking 222 having been intersected (e.g., bisected) to form the respective cut end 218 of the respective fiber tow 214. The markings applied at 104 may be any desired shape or pattern. While Figs. 5-6 illustrate circular shaped markings 222 that are cut in half to form semi-circles when the fiber tow is cut, the present disclosure is not limited to the same, and may include markings 222 that are oval, ellipsoid, square, triangle, rectangle, and/or diamond shaped, and/or markings 222 may include stripes, dots, dashes, lines, letters, numbers, encoded data (e.g., QR codes or other barcodes), etc.

In some methods 100, applying the at least one marking at 104 includes applying at least one marking in a plurality of different locations along the widths of the fiber tows. For example, with reference again to Fig. 4, each fiber tow 214 has a respective width 234 extending substantially parallel to cut end 218 of the respective fiber tow 214. The markings 222 of this example have been applied at different respective locations along the widths 234 of the respective fiber tows 214, such that the respective location of the respective marking 222 along width 234 may be configured to identify a lane of the automated lamination machine from with the respective fiber tow 214 was placed. For example, fiber tow 214a has a marking 222a near the middle of its width 234, which may indicate that fiber tow 214a was placed by a first lane of the automated lamination machine, fiber tow 214b has a marking 222b about a third of its width 234 away from a side edge 226b of fiber tow 214b, which may indicate that fiber tow 214b was placed by a second lane of the automated lamination machine, and fiber tow 214c has a marking 222c which is placed along its width 234 nearer to top edge 226c of fiber tow 214c, which may indicate that fiber tow 214c was placed by a third lane of the automated lamination machine. The correspondence between the positions of the markings and the lanes of the automated lamination machine may be set by the control program.

Once the material is laid down, or as it is laid down, the ends of each fiber tow and/or the fiber direction can be inspected, at 106, using the markings applied to the fiber tows, such as to inspect the ply boundaries during lamination. Thus, methods 100 also may include visually inspecting the fiber tows at 106, such as by examining or analyzing the marking of each respective fiber tow (e.g., each marking 222 of each fiber tow 214). In some methods 100, visually inspecting the fiber tows at 106 includes manually inspecting the markings on the fiber tows. Additionally or alternatively, visually inspecting the fiber tows at 106 may include automated inspecting, automated image analysis, and/or artificial intelligence (AI) inspection of the markings (e.g., by an image analysis device 230, which may be or include a camera system, a computer vision system, and/or an AI image analysis system). To these ends, applying the markings at 104 may include applying at least one marking configured to be interpreted by the human eye, and/or applying at least one marking configured to be interpreted by machine. Disclosed methods 100 may enable higher rates of inspection of composite parts as they are being built as compared to prior art techniques. Additionally or alternatively, disclosed methods 100 and systems 202 may be configured to combine cutting of fiber tows (at 102), marking of fiber tows (at 104), and image analysis or other inspection of the fiber tows (at 106).

In some examples, visually inspecting the markings at 106 includes identifying a respective location of each respective cut end (e.g., a respective location of each cut end 218 of each fiber tow 214), and/or determining a respective fiber direction of each respective fiber tow of the plurality of fiber tows. Visually inspecting at 106 may be performed as the automated lamination machine lays down each respective fiber tow and may include determining a tow gap 232 between a first respective fiber tow and a previously-placed respective fiber tow. In some examples, a subsequently-placed respective fiber tow is placed. For example, and as illustrated in Fig. 4, a tow gap 232 between fiber tow 214b and previously placed fiber tow 214a may be determined at the visual inspection step 106, optionally before subsequently placed fiber tow 214c is laid down next to fiber tow 214b. Visually inspecting the fiber tows at 106 may be repeated for each respective fiber tow laid down in a given part or structure, and/or for a plurality of plies of fiber tows.

The markings may enable determination of a tow gap, also referred to herein as a tow-to-tow distance, gap, or spacing, and/or course-to-course gaps of the material as it is laid down, without requiring the use of a laser profilometer or similar. For example, applying the markings at 104 may include marking a first fiber tow (e.g., fiber tow 214a in Fig. 4) and marking a second fiber tow (e.g., fiber tow 214b), and methods 100 may further include determining a tow-to-tow spacing (e.g., tow gap 232 between fiber tow 214a and fiber tow 214b) between the first fiber tow and the second fiber tow, based on the first marking and the second marking, at 110. Determining the tow-to-tow spacing at 110 may be performed automatically (e.g., by image analysis device 230), and may be performed for each respective pair of adjacent fiber tows that are placed by the automated lamination machine or may be performed for a subset of such pairs of adjacent fiber tows. In some examples, the adjacent pairs of fiber tows are substantially parallel to one another. In some examples, determining the tow-to-tow spacing at 110 includes determining a mark-to-mark distance between the first marking of the first fiber tow (e.g., marking 222a of fiber tow 214a) and the second marking of the second fiber tow (e.g., marking 222b of fiber tow 214b). Additionally or alternatively, determining the tow-to-tow spacing at 110 may include determining a distance between a first side edge 226 of a first fiber tow (e.g., side edge 226a of fiber tow 214a) and a second side edge of the second fiber tow (e.g., side edge 226b of fiber tow 214b), with the first side edge 226 of the first fiber tow optionally being substantially perpendicular to the cut end 218 of the first fiber tow, and the second side edge 226 of the second fiber tow optionally being substantially perpendicular to the cut end 218 of the second fiber tow. Determining the tow-to-tow spacing at 110 also may include determining whether a side edge of the first fiber tow overlaps a side edge of the second fiber tow.

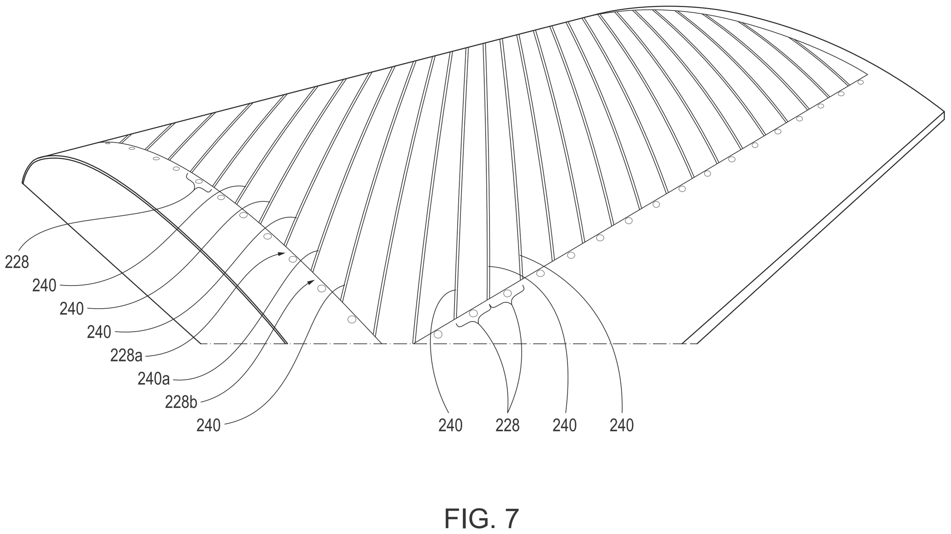

Methods 100 may include determining a course gap between the first fiber tow and the second fiber tow, based on the first marking and the second marking, at 112. For example, Fig. 7 illustrates a plurality of courses 228 of material (e.g., each course 228 consisting of a plurality of fiber tows 214) placed on a mold, or tooling, 238. Course gaps 240 between adjacent courses 228 (e.g., course gap 240a between course 228a and course 228b) may be determined at 112 based on the markings applied to the respective fiber tows of the different courses at 104. In some examples, different markings may be applied at 104 to respective fiber tows forming an edge of a course (sometimes referred to as "edge tows") to facilitate course gap location and inspection. For example, Fig. 4 shows an example of markings 222 that may be used for individual fiber tow identification and/or for tow-to-tow spacing determination, and course gap markings 242 (which are an example of markings 222) that may be used for course gap determination. Some fiber tows 214 may have both markings 222 and course gap markings 242, as shown in Fig. 4. In some examples, the markings applied at 104 may be configured to distinguish between the respective cut end of a respective fiber tow and a respective side edge of the respective fiber tow.

Applying the markings at 104 and visually inspecting the markings at 106 may be used to create a feedback loop for quality control or preventative maintenance of systems such as system 202 (Fig.2). For example, feedback may be provided to the automated lamination machine at 108, based on inspection of the applied markings, to adjust future placement of fiber tows at 102 and/or future markings being applied at 104. Additionally or alternatively, methods 100 may include storing data at 114, such as storing the results of visually inspecting the markings at 106, storing the tow gaps determined at 110, and/or storing the course gaps determined at 112. Additionally or alternatively, storing data at 114 may include identifying each individual tow laid down by the automated lamination machine and retaining this information and/or storing a digital twin of each fiber tow laid down by the automated lamination machine.

In some examples, applying the markings at 104 includes cuing a vision system (e.g., image analysis device 230) by applying a visual cue and/or control point configured to be recognized by a camera system, a computer vision system, and/or an AI image analysis system (e.g., image analysis device 230), with the visual cue being configured to trigger the camera system, the computer vision system, and/or the AI image analysis system to visually inspect the respective fiber tow on which the visual cue is placed. In some examples, a respective visual cue may be applied to each respective fiber tow, while in some examples, a respective visual cue may be applied to a subset of respective fiber tows amongst the material laid down by the automated lamination machine. In a specific example, a series of dots or other shapes may be arranged in a specific pattern to cue the vision system to inspect the fiber tow on which the cue appears, and/or may direct the vision system to inspect a particular location of the fiber tow. In some examples, the visual cue can direct the vision system to inspect the fiber tows at a certain predetermined distance from the cut end of the fiber tow, and/or at a certain predetermined distance from the visual cue itself. In various examples, applying the markings at 104 may include applying a first portion of markings that are configured to give location information about the respective fiber tow on which the marking is placed, and applying a second portion of markings that are configured to cue the vision system to look for other markings and/or characteristics about the fiber tow's placement. In some examples, the same markings may be used for both location information and cuing the camera system, while in some examples, different markings may be used for different purposes.

Any suitable material may be used in laying down the fiber tows in disclosed methods 100, including but not limited to dry fiber non-crimp fabric material for resin infusion, thermoset prepreg materials, and/or thermoplastic prepreg materials.

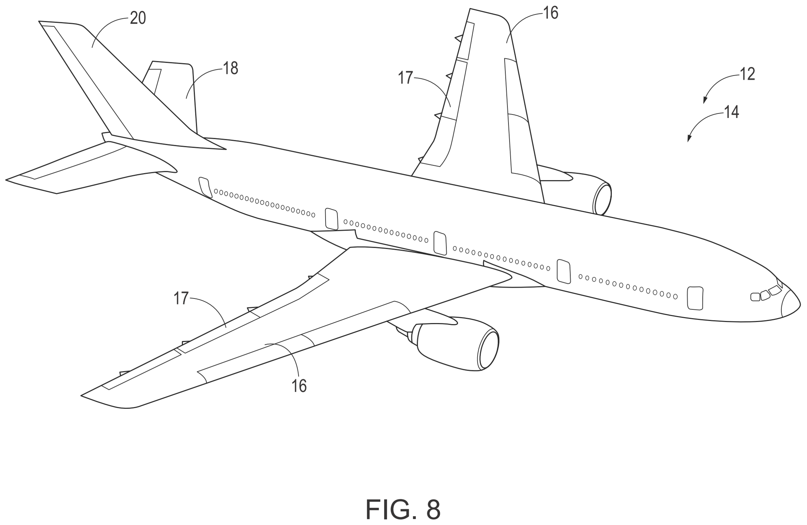

Disclosed methods 100 and/or systems 202 may be used in the manufacture of any desired composite parts. With reference to Fig. 8, one or more composite parts made with markings as described herein may be included in an apparatus 12. Composite parts may be utilized in many different industries and applications, such as the aerospace, automotive, architecture, marine, wind power generation, remote control aircraft, military, recreation, and/or motorsport industries. In Fig. 8, an example of apparatus 12 that may include one or more composite parts with markings as described herein generally is illustrated in the form of an aircraft 14. Aircraft 14 may take any suitable form, including commercial aircraft, military aircraft, or any other suitable aircraft. While Fig. 8 illustrates aircraft 14 in the form of a fixed-wing aircraft, other types and configurations of aircraft are within the scope of aircraft 14 according to the present disclosure, including (but not limited to) rotorcraft and helicopters.

Apparatus 12 (e.g., aircraft 14) may include one or more composite parts made according to methods of the present disclosure. As illustrative, non-exclusive examples, composite parts may be utilized in wings 16 (e.g., flaps 17, which may be inboard or outboard flaps), though other components of aircraft 14, such as horizontal stabilizers 18, vertical stabilizers 20, and other components additionally or alternatively may include one or more composite parts. Other applications in aircraft 14 (or other apparatus 12) for composite parts made according to presently disclosed methods 100 may include other wing control surfaces, ailerons, flaperons, air brakes, elevators, slats, spoilers, rudders, canards, and/or winglets. In other industries, examples of apparatus 12 including one or more composite parts may include or be a portion of space satellites, transit vehicles, shipping containers, rapid transit vehicles, automobile bodies, propeller blades, turbine blades, and/or marine vehicles (e.g., sailboats), among others.

Illustrative, non-exclusive examples of inventive subject matter according to the present disclosure are described in the following enumerated paragraphs:
A1. A method (100), comprising:
   laying down (102) a plurality of fiber tows (214), wherein the laying down (102) the plurality of fiber tows (214) is performed via an automated lamination machine (200), wherein each respective fiber tow (214) of the plurality of fiber tows (214) comprises a respective cut end (218); and
   applying (104) at least one marking (222) within at least one marked region (224) of each respective fiber tow (214) of the plurality of fiber tows (214), wherein each respective marking (222) is configured to provide location information about the respective fiber tow (214) on which the respective marking (222) is placed, and wherein the at least one marking (222) is configured to enable visual inspection of the plurality of fiber tows (214), optionally wherein the applying (104) the at least one marking (222) comprises applying a contrasting color onto each respective fiber tow (214) of the plurality of fiber tows (214), wherein the contrasting color is configured to contrast with a color of the fiber tows (214) such that the at least one marking (222) is configured to enable visual inspection of the plurality of fiber tows (214)..
A1.1. The method (100) of paragraph A1, wherein at least one marked region (224) is positioned at or adjacent to the respective cut end (218) of the respective fiber tow (214).
A1.2. The method (100) of paragraph A1 or A1.1, wherein at least one marked region (224) is positioned at or adjacent to a respective side edge (226) of the respective fiber tow (214), wherein the respective side edge (226) is substantially perpendicular to the respective cut end (218) of the respective fiber tow (214).
A1.3. The method (100) of any of paragraphs A1-A1.2, wherein the applying (104) the at least one marking (222) is automated.
A1.4. The method (100) of any of paragraphs A1-A1.3, wherein the applying (104) the at least one marking (222) is performed by the automated lamination machine (200), optionally wherein the applying (104) the at least one marking (222) is performed by the automated lamination machine (200) during the laying down (102) the plurality of fiber tows (214).
A1.5. The method (100) of any of paragraphs A1-A1.4, further comprising causing the automated lamination machine (200) to perform the laying down (102) the plurality of fiber tows (214) according to an engineering definition for a composite part to be formed by the plurality of fiber tows (214).
A1.6. The method (100) of any of paragraphs A1-A1.5, wherein the automated lamination machine (200) comprises an automated fiber placement (AFP) machine and/or a contoured tape laying machine (CTLM).
A2. The method (100) of any of paragraphs A1-A1.5, wherein the laying down (102) the plurality of fiber tows (214) comprises laying down the plurality of fiber tows (214) such that the plurality of fiber tows (214) at least partially overlaps an underlying layer.
A3. The method (100) of paragraph A2, wherein the at least one marking (222) is configured to highlight and differentiate each respective fiber tow (214) from the underlying layer.
A3.1. The method (100) of any of paragraphs A1-A3, wherein the at least one marking (222) is configured to highlight and differentiate each respective fiber tow (214) from one or more adjacent fiber tows (214) of the plurality of fiber tows (214).
A4. The method (100) of any of paragraphs A1-A3.1, wherein the applying (104) the at least one marking (222) comprises spraying the at least one marking (222) on each respective fiber tow (214) of the plurality of fiber tows (214).
A5. The method (100) of any of paragraphs A1-A4, wherein the applying (104) the at least one marking (222) comprises applying contrasting materials that are able to come in contact with an un-cured resin without chemically reacting with the un-cured resin, and wherein the plurality of fiber tows (214) are pre-impregnated with the un-cured resin.
A6. The method (100) of any of paragraphs A1-A5, wherein each respective cut end (218) of each respective fiber tow (214) of the plurality of fiber tows (214) is located within the at least one marked region (224) of the respective fiber tow (214).
A7. The method (100) of any of paragraphs A1-A6, further comprising visually inspecting (106) the plurality of fiber tows (214) by examining or analyzing the at least one marking (222) of each respective fiber tow (214).
A8. The method (100) of paragraph A7, wherein the visually inspecting (106) comprises manually inspecting.
A8.1. The method (100) of any of paragraphs A1-A8, wherein the applying (104) the at least one marking (222) comprises applying at least one marking (222) configured to be interpreted by the human eye.
A9. The method (100) of any of paragraphs A7-A8.1, wherein the visually inspecting (106) comprises automated inspecting.
A9.1. The method (100) of any of paragraphs A7-A9, wherein the visually inspecting (106) comprises automated image analysis.
A9.2. The method (100) of any of paragraphs A7-A9.1, wherein the visually inspecting (106) comprises artificial intelligence (AI) inspection.
A10. The method (100) of any of paragraphs A7-A9.2, wherein the visually inspecting (106) comprises identifying a respective location of each respective cut end (218).
A11. The method (100) of any of paragraphs A7-A10, wherein the visually inspecting (106) comprises determining a respective fiber direction of each respective fiber tow (214) of the plurality of fiber tows (214).
A11.1. The method (100) of any of paragraphs A7-A11, wherein the visually inspecting (106) is performed as the automated lamination machine (200) lays down each respective fiber tow (214) of the plurality of fiber tows (214).
A11.2. The method (100) of any of paragraphs A7-A11.1, wherein the visually inspecting (106) comprises determining (110) a tow gap (232) between a first respective fiber tow (214) of the plurality of fiber tows (214) and a previously-placed respective fiber tow (214) of the plurality of fiber tows (214).
A11.3. The method (100) of paragraph A11.2, wherein the tow gap (232) is determined before a subsequently-placed respective fiber tow (214) of the plurality of fiber tows (214) is placed.
A11.4. The method (100) of any of paragraphs A7-A11.3, wherein the visually inspecting (106) is repeated for each respective fiber tow (214) of the plurality of fiber tows (214).
A11.5. The method (100) of any of paragraphs A7-A11.4, wherein the visually inspecting (106) is repeated for a plurality of layers of fiber tows (214).
A11.6. The method (100) of any of paragraphs A1-A11.5, wherein the applying (104) the at least one marking (222) comprises applying at least one marking (222) configured to be interpreted by a machine.
A12. The method (100) of any of paragraphs A1-A11.6, wherein the applying (104) the at least one marking (222) is performed by an end effector (204) of the automated lamination machine (200) in an automated fashion.
A13. The method (100) of paragraph A12, wherein the applying (104) the at least one marking (222) is automatically performed as each respective fiber tow (214) of the plurality of fiber tows (214) is laid down.
A13.1. The method (100) of any of paragraphs A1-A13, wherein the applying (104) the at least one marking (222) is performed before material (208) used to form the plurality of fiber tows (214) is placed onto a spool or creel (210) configured for use with the automated lamination machine (200).
A14. The method (100) of any of paragraphs A1-A13.1, wherein the applying (104) the at least one marking (222) comprises spraying, inking, rolling on, and/or otherwise applying a visual indicator of a contrasting color onto each respective fiber tow (214) of the plurality of fiber tows (214), wherein the contrasting color is configured to contrast with an/the underlying layer of material (208).
A15. The method (100) of any of paragraphs A1-A14, wherein the applying (104) the at least one marking (222) comprises color striping.
A16. The method (100) of any of paragraphs A1-A15, wherein the applying (104) the at least one marking (222) comprises applying a QR code.
A16.1. The method (100) of paragraph A16, wherein the QR code is configured to directly connect placement of each respective fiber tow (214) of the plurality of fiber tows (214) to one or more of inspection reports and documentation.
A17. The method (100) of any of paragraphs A1-A16.1, wherein the laying down (102) the plurality of fiber tows (214) comprises cutting each fiber tow (214) of the plurality of fiber tows (214) from a/the spool or creel (210) of material (208), thereby forming each respective cut end (218) of each respective fiber tow (214) of the plurality of fiber tows (214).
A18. The method (100) of any one of paragraphs A1-A17, wherein the applying (104) the at least one marking (222) comprises:
   identifying a respective location of each respective cut end (218) on the material (208); and
   applying each respective marking (222) of the at least one marking (222) to the material (208) such that each respective marking (222) intersects a respective cut end (218) when the material (208) is cut.
A19. The method (100) of paragraph A18, wherein the applying each respective marking (222) comprises applying a shaped marking (222) to the material (208) at each respective location of each respective cut end (218).
A19.1 The method (100) of paragraph A19, wherein the applying the shaped marking (222) comprises applying a circular marking (222).
A19.2. The method (100) of paragraph A19.1, wherein the circular marking (222) is bisected by the respective cut end (218).
A20. The method (100) of any of paragraphs A1-A19.2, wherein each fiber tow (214) of the plurality of fiber tows (214) has a respective width (234) extending substantially parallel to the respective cut end (218) of the respective fiber tow (214), and wherein the applying (104) the at least one marking (222) comprises applying the at least one marking (222) in a plurality of different locations along the respective width (234) of the respective fiber tow (214).
A21. The method (100) of any of paragraphs A1-A20, wherein each fiber tow (214) of the plurality of fiber tows (214) has a/the respective width (234) extending substantially parallel to the cut end (218) of the respective fiber tow (214), and wherein the applying (104) the at least one marking (222) comprises applying the at least one marking (222) at a respective location along the respective width (234) of the respective fiber tow (214), such that the respective location along the respective width (234) of the respective fiber tow (214) is configured to identify a lane from which the respective fiber tow (214) was placed.
A22. The method (100) of any of paragraphs A1-A21, wherein the applying (104) the at least one marking (222) is configured to create a feedback loop for quality control or preventative maintenance.
A23. The method (100) of any of paragraphs A1-A22, wherein the applying (104) the at least one marking (222) comprises applying a different respective marking (222) to each respective fiber tow (214).
A24. The method (100) of paragraph A23, wherein the applying (104) the at least one marking (222) comprises applying a different respective QR code to each respective fiber tow (214).
A25. The method (100) of any of paragraphs A1-A24, wherein the applying (104) the at least one marking (222) within the at least one marked region (224) of each respective fiber tow (214) comprises:
   applying a first marking (222a) to a first fiber tow (214a) of the plurality of fiber tows (214); and
   applying a second marking (222b) to a second fiber tow (214b) of the plurality of fiber tows (214).
A26. The method (100) of paragraph A25, wherein the method (100) further comprises determining (110) a tow-to-tow spacing (232) between the first fiber tow (214a) and the second fiber tow (214b), based on the first marking (222a) and the second marking (222b).
A27. The method (100) of paragraph A26, wherein the first fiber tow (214a) and the second fiber tow (214b) are adjacent to one another and substantially parallel with one another.
A28. The method (100) of paragraph A26 or A27, wherein the determining (110) the tow-to-tow spacing (232) is performed without a laser profilometer or similar.
A29. The method (100) of any of paragraphs A26-A28, wherein the determining (110) the tow-to-tow spacing (232) comprises determining a mark-to-mark distance between the first marking (222a) and the second marking (222b).
A30. The method (100) of any of paragraphs A26-A29, wherein the determining (110) the tow-to-tow spacing (232) comprises determining a distance between a first side edge (226a) of the first fiber tow (214a) and a second side edge (226b) of the second fiber tow (214b), wherein the first side edge (226a) of the first fiber tow (214a) is substantially perpendicular to the cut end (218) of the first fiber tow (214a), and wherein the second side edge (226b) of the second fiber tow (214b) is substantially perpendicular to the cut end (218) of the second fiber tow (214b).
A31. The method (100) of any of paragraphs A26-A30, wherein the determining the tow-to-tow spacing (232) comprises determining whether a/the first side edge (226a) of the first fiber tow (214a) overlaps a/the second side edge (226b) of the second fiber tow (214b).
A32. The method (100) of any of paragraphs A25-A31, wherein the method (100) further comprises determining a course gap (240) between the first fiber tow (214a) and the second fiber tow (214b), based on the first marking (222a) and the second marking (222b).
A33. The method (100) of any of paragraphs A1-A32, wherein the applying (104) the at least one marking (222) is configured to distinguish between the respective cut end (218) of the respective fiber tow (2140) and a/the respective side edge (226) of the respective fiber tow (214).
A34. The method (100) of any of paragraphs A1-A33, wherein the plurality of fiber tows (214) comprise dry fiber non-crimp fabric material (208) for resin infusion.
A35. The method (100) of any of paragraphs A1-A33, wherein the plurality of fiber tows (214) comprise thermoset prepreg materials.
A36. The method (100) of any of paragraphs A1-A33, wherein the plurality of fiber tows (214) comprise thermoplastic prepreg materials.
A37. The method (100) of any of paragraphs A1-A36, wherein the applying (104) the at least one marking (222) comprises applying a visual cue configured to be recognized by an image analysis device (230) such as a camera system, a computer vision system, and/or an AI image analysis system, and wherein the visual cue is configured to trigger the camera system, the computer vision system, and/or the AI image analysis system to visually inspect the respective fiber tow on which the visual cue is placed.
A38. The method (100) of paragraph A37, wherein the applying the visual cue comprises applying a respective visual cue to each respective fiber tow (214) of the plurality of fiber tows (214).
A39. The method (100) of any of paragraphs A1-A38, wherein at least a first portion of the at least one marking (222) is configured to provide location information about the respective fiber tow (214) on which the at least one marking (222) is placed.
A40. The method (100) of any of paragraphs A1-A39, wherein at least a second portion of the at least one marking (222) comprises a visual cue configured to be recognized by a camera system, a computer vision system, and/or an AI image analysis system.
A41. The method (100) of any of paragraphs A1-A40, further comprising cuing (104) a/the camera system, a/the computer vision system, and/or a/the AI image analysis system via a/the visual cue, thereby triggering the camera system, the computer vision system, and/or the AI image analysis system to visually inspect the respective fiber tow (214) on which the visual cue is placed.
A42. A method (100), comprising:
   laying down (102) a plurality of fiber tows (214), wherein the laying down (102) the plurality of fiber tows (214) is performed via an automated lamination machine (200), wherein each respective fiber tow (214) of the plurality of fiber tows (214) comprises a respective cut end (218), and wherein the plurality of fiber tows (214) are pre-impregnated with un-cured resin;
   applying (104) at least one marking (222) within at least one marked region (224) of each respective fiber tow (214) of the plurality of fiber tows (214), wherein at least a first portion of the at least one marking (222) is configured to provide location information about the respective fiber tow (214) on which the at least one marking (222) is placed, wherein the applying (104) the at least one marking (222) comprises applying a contrasting color onto each respective fiber tow (214) of the plurality of fiber tows (214), wherein the contrasting color is configured to contrast with a color of the fiber tows (214) such that the at least one marking (222) is configured to enable visual inspection of the plurality of fiber tows (214), wherein the applying (104) the at least one marking (222) is performed automatically by the automated lamination machine (200) during the laying down (102) the plurality of fiber tows (214), and wherein at least a second portion of the at least one marking (222) comprises a visual cue configured to be recognized by a camera system, a computer vision system, and/or an AI image analysis system;
   cuing (104) the camera system, the computer vision system, and/or the AI image analysis system via the visual cue, thereby triggering the camera system, the computer vision system, and/or the AI image analysis system to visually inspect the respective fiber tow (214) on which the visual cue is placed; and
   visually inspecting (106) the plurality of fiber tows (214) by examining or analyzing the marking (222) of each respective fiber tow (214) to identify a respective location of each respective cut end (218) of each of the plurality of fiber tows (214), wherein the visually inspecting (106) is performed as the automated lamination machine (200) lays down each respective fiber tow (214) of the plurality of fiber tows (214), such that the visually inspecting (106) comprises determining a gap (232) between a first respective fiber tow (214) of the plurality of fiber tows (214) and a previously-placed respective fiber tow (214) of the plurality of fiber tows (214) before a subsequently-placed respective fiber tow (214) of the plurality of fiber tows (214) is placed.
B1. A system (202) comprising an automated lamination machine (200) adapted to perform the method (100) of any of paragraphs A1-A42.
B2. The system (202) of paragraph B1, wherein the system (202) is configured to cut each respective fiber tow (214) of the plurality of fiber tows (214) from a/the spool or creel (210) of material (208).
B3. The system (202) of paragraph B1 or B2, wherein the system (202) is configured to apply at least one mark (222) to each respective fiber tow (214) of the plurality of fiber tows (214).
B4. The system (202) of any of paragraphs B1-B3, wherein the system (202) is configured to visually inspect each respective fiber tow (214) of the plurality of fiber tows (214), using the at least one marking (222) of each respective fiber tow (214).
C1. The use of an automated fiber placement (AFP) machine to perform the method (100) of any of paragraphs A1-A42.
C2. The use of a contoured tape laying machine (CTLM) to perform the method (100) of any of paragraphs A1-A42.

As used herein, the terms "selective" and "selectively," when modifying an action, movement, configuration, or other activity of one or more components or characteristics of an apparatus, mean that the specific action, movement, configuration, or other activity is a direct or indirect result of dynamic processes and/or user manipulation of an aspect of, or one or more components of, the apparatus. The terms "selective" and "selectively" thus may characterize an activity that is a direct or indirect result of user manipulation of an aspect of, or one or more components of, the apparatus, or may characterize a process that occurs automatically, such as via the mechanisms disclosed herein.

As used herein, the terms "adapted" and "configured" mean that the element, component, or other subject matter is designed and/or intended to perform a given function. Thus, the use of the terms "adapted" and "configured" should not be construed to mean that a given element, component, or other subject matter is simply "capable of" performing a given function but that the element, component, and/or other subject matter is specifically selected, created, implemented, utilized, programmed, and/or designed for the purpose of performing the function. It is also within the scope of the present disclosure that elements, components, and/or other recited subject matter that is recited as being adapted to perform a particular function may additionally or alternatively be described as being configured to perform that function, and vice versa. Similarly, subject matter that is recited as being configured to perform a particular function may additionally or alternatively be described as being operative to perform that function.

As used herein, the phrase "at least one," in reference to a list of one or more entities should be understood to mean at least one entity selected from any one or more of the entities in the list of entities, but not necessarily including at least one of each and every entity specifically listed within the list of entities and not excluding any combinations of entities in the list of entities. This definition also allows that entities may optionally be present other than the entities specifically identified within the list of entities to which the phrase "at least one" refers, whether related or unrelated to those entities specifically identified. Thus, as a non-limiting example, "at least one of A and B" (or, equivalently, "at least one of A or B," or, equivalently "at least one of A and/or B") may refer, in one example, to at least one, optionally including more than one, A, with no B present (and optionally including entities other than B); in another example, to at least one, optionally including more than one, B, with no A present (and optionally including entities other than A); in yet another example, to at least one, optionally including more than one, A, and at least one, optionally including more than one, B (and optionally including other entities). In other words, the phrases "at least one," "one or more," and "and/or" are open-ended expressions that are both conjunctive and disjunctive in operation. For example, each of the expressions "at least one of A, B, and C," "at least one of A, B, or C," "one or more of A, B, and C," "one or more of A, B, or C" and "A, B, and/or C" may mean A alone, B alone, C alone, A and B together, A and C together, B and C together, or A, B, and C together, and optionally any of the above in combination with at least one other entity.

As used herein, the phrase "at least substantially," when modifying a degree or relationship, includes not only the recited "substantial" degree or relationship, but also the full extent of the recited degree or relationship. A substantial amount of a recited degree or relationship may include at least 75% of the recited degree or relationship. For example, a first direction that is at least substantially parallel to a second direction includes a first direction that is within an angular deviation of 22.5° relative to the second direction and also includes a first direction that is identical to the second direction.

The various disclosed elements of apparatuses and steps of methods disclosed herein are not required to all apparatuses and methods according to the present disclosure, and the present disclosure includes all novel and non-obvious combinations and subcombinations of the various elements and steps disclosed herein. Moreover, one or more of the various elements and steps disclosed herein may define independent inventive subject matter that is separate and apart from the whole of a disclosed apparatus or method. Accordingly, such inventive subject matter is not required to be associated with the specific apparatuses and methods that are expressly disclosed herein, and such inventive subject matter may find utility in apparatuses and/or methods that are not expressly disclosed herein.

As used herein, the phrase, "for example," the phrase, "as an example," and/or simply the term "example," when used with reference to one or more components, features, details, structures, examples, and/or methods according to the present disclosure, are intended to convey that the described component, feature, detail, structure, example, and/or method is an illustrative, non-exclusive example of components, features, details, structures, examples, and/or methods according to the present disclosure. Thus, the described component, feature, detail, structure, example, and/or method is not intended to be limiting, required, or exclusive/exhaustive; and other components, features, details, structures, examples, and/or methods, including structurally and/or functionally similar and/or equivalent components, features, details, structures, examples, and/or methods, are also within the scope of the present disclosure.

## Claims

1. A method (100), comprising:
laying down (102) a plurality of fiber tows (214), wherein the laying down (102) the plurality of fiber tows (214) is performed via an automated lamination machine (200), wherein each respective fiber tow (214) of the plurality of fiber tows (214) comprises a respective cut end (218); and
applying (104) at least one marking (222) within at least one marked region (224) of each respective fiber tow (214) of the plurality of fiber tows (214), wherein the at least one marking (222) is configured to provide location information about the respective fiber tow (214) on which the at least one marking (222) is placed, and wherein the applying (104) the at least one marking (222) comprises applying a contrasting color onto each respective fiber tow (214) of the plurality of fiber tows (214), wherein the contrasting color is configured to contrast with a color of the fiber tows (214) such that the at least one marking (222) is configured to enable visual inspection of the plurality of fiber tows (214).

2. The method (100) according to claim 1, wherein at least one marked region (224) is positioned at or adjacent to the respective cut end (218) of the respective fiber tow (214), optionally wherein the applying (104) the at least one marking (222) is performed automatically by the automated lamination machine (200) during the laying down (102) the plurality of fiber tows (214).

3. The method (100) according to claim 1 or 2, wherein the laying down (102) the plurality of fiber tows (214) comprises laying down the plurality of fiber tows (214) such that the plurality of fiber tows (214) at least partially overlaps an underlying layer, and wherein the at least one marking (222) is configured to highlight and differentiate each respective fiber tow (214) from the underlying layer.

4. The method (100) according to any one of the preceding claims, wherein the at least one marking (222) is configured to highlight and differentiate each respective fiber tow (214) from one or more adjacent fiber tows (214) of the plurality of fiber tows (214).

5. The method (100) according to any one of the preceding claims, wherein the applying (104) the at least one marking (222) comprises applying contrasting materials that are able to come in contact with an un-cured resin without chemically reacting with the un-cured resin, and wherein the plurality of fiber tows (214) is pre-impregnated with the un-cured resin.

6. The method (100) according to any one of the preceding claims, wherein the applying (104) the at least one marking (222) comprises applying at least one marking (222) configured to be interpreted by the human eye, optionally wherein the applying (104) the at least one marking (222) comprises applying at least one marking (222) configured to be interpreted by a machine.

7. The method (100) according to any one of the preceding claims, further comprising visually inspecting (106) the plurality of fiber tows (214) by examining or analyzing the at least one marking (222) of each respective fiber tow (214), optionally wherein the visually inspecting (106) comprises identifying a respective location of each respective cut end (218).

8. The method (100) according to claim 7, wherein the visually inspecting (106) is performed as the automated lamination machine (200) lays down each respective fiber tow (214) of the plurality of fiber tows (214), such that the visually inspecting (106) comprises determining a gap (232) between a first respective fiber tow (214) of the plurality of fiber tows (214) and a previously-placed respective fiber tow (214) of the plurality of fiber tows (214) before a subsequently-placed respective fiber tow (214) of the plurality of fiber tows (214) is placed, optionally wherein the visually inspecting (106) is repeated for each respective fiber tow (214) of the plurality of fiber tows (214).

9. The method (100) according to any one of the preceding claims, wherein the applying (104) the at least one marking (222) comprises applying a QR code, wherein the QR code is configured to directly connect placement of each respective fiber tow (214) of the plurality of fiber tows (214) to one or more of inspection reports and documentation.

10. The method (100) according to any one of the preceding claims, wherein the applying (104) the at least one marking (222) comprises:
identifying a respective location of each respective cut end (218) on a material (208); and
applying each respective marking (222) of the at least one marking (222) to the material (208) such that each respective marking (222) intersects a respective cut end (218) when the material (208) is cut.

11. The method (100) according to any one of the preceding claims, wherein the applying (104) the at least one marking (222) is configured to create a feedback loop for quality control or preventative maintenance.

12. The method (100) according to any one of the preceding claims, wherein the applying (104) the at least one marking (222) within the at least one marked region (224) of each respective fiber tow (214) comprises:
applying a first marking (222a) to a first fiber tow (214a) of the plurality of fiber tows (214);
applying a second marking (222b) to a second fiber tow (214b) of the plurality of fiber tows (214);
determining a tow-to-tow spacing (232) between the first fiber tow (214a) and the second fiber tow (214b), based on the first marking (222a) and the second marking (222b), wherein the first fiber tow (214a) and the second fiber tow (214b) are adjacent to one another and substantially parallel with one another, and wherein the determining the tow-to-tow spacing (232) is performed without a laser profilometer.

13. The method (100) according to claim 12, wherein the determining the tow-to-tow spacing (232) comprises determining whether a first side edge (226a) of the first fiber tow (214a) overlaps a second side edge (226b) of the second fiber tow (214b).

14. The method (100) of according to claim 12 or 13, wherein the method (100) further comprises determining (112) a course gap (240) between the first fiber tow (214a) of the plurality of fiber tows (214) and the second fiber tow (214b) of the plurality of fiber tows (214), based on the first marking (222a) and the second marking (222b).

15. The method (100) according to claim 1, wherein the applying (104) the at least one marking (222) comprises applying a visual cue configured to be recognized by a camera system, a computer vision system, and/or an AI image analysis system, wherein the visual cue is configured to trigger the camera system, the computer vision system, and/or the AI image analysis system to visually inspect the respective fiber tow (214) on which the visual cue is placed, and wherein the applying the visual cue comprises applying a respective visual cue to each respective fiber tow (214) of the plurality of fiber tows (214).
